# EUROPEAN PATENT APPLICATION

(11) **EP 1 992 601 A1**
(43) Date of publication of application: **19.11.2008**
(21) Application number: 07251955.6
(22) Date of filing: 11.05.2007
(51) Int. Cl.: C07C 1/24, C07C 11/04

(54) **Dehydration of alcohols over supported heteropolyacids**

(71) Applicant: INEOS EUROPE LIMITED, Lyndhurst, Hampshire, SO43 7FG (GB)
(72) Inventor: The designation of the inventor has not yet been filed
(74) Representative: King, Alex

(57) **Abstract**

The present invention relates to a process for the dehydration of one or more alcohols, and in particular to a process for the dehydration of one or more alcohols, which process comprises contacting one or more alcohols with a supported heteropolyacid catalyst, said supported heteropolyacid catalyst having a heteropolyacid loading of from 0.5 to 0.9 mg/m² of catalyst support surface area.

## Description

The present invention relates to a process for the dehydration of alcohols to the corresponding olefins, and in particular, using heteropolyacid catalysts.

The dehydration of alcohols to olefins catalysed using a wide variety of acid catalysts is well-known.

WO 2007/003899 and WO 2007/003901, for example, describe reactive distillation processes for the dehydration of mixed alcohols. The preferred catalysts are heteropolyacids, most preferably impregnated on a support at a loading of 30 to 50 wt% based on the total weight of heteropolyacid and support. For a heteropolyacid support with a surface area of 315 m²/g (typical for Grace 57 silica which is one of the preferred supports in WO 2007/03899 and WO 2007/003901), this range corresponds to a loading of approximately 1.4 to 3.2 mg/m² of catalyst support surface area.

It has now been found that significant advantages are found in alcohol dehydration by supported heteropolyacid catalysts by selection of specific catalyst loadings. In particular, it has surprisingly been found that over a relatively narrow range of loadings improved ethylene productivity and reduced carbon formation on the catalyst is obtained.

Accordingly, in a first aspect the present invention provides a process for the dehydration of one or more alcohols, which process comprises contacting one or more alcohols with a supported heteropolyacid catalyst, said supported heteropolyacid catalyst having a heteropolyacid loading of from 0.5 to 0.9 mg/m² of catalyst support surface area.

Preferably, the one or more alcohols is a mixture of alcohols, especially comprising ethanol and propanol. Higher alcohols may also be present, but preferably the heaviest alcohols are C6 alcohols.

The one or more alcohols may be provided as any suitable feed comprising said alcohols. The feed may also comprise one or more ethers, such as diethyl ether, dipropyl ether and ethyl propyl ether.

The feed may also comprise water, typically in an amount of less than 10wt%, for example 1-7wt%.

Any suitable heteropolyacid may be used. The heteropolyacid may be used in the form of a free acid or may be used in the form of a heteropolyacid salt. The heteropolyacid anion is a complex, high molecular weight entity and, typically, the anion comprises 2-18 oxygen-linked polyvalent metal atoms, which are called peripheral atoms. These peripheral atoms surround one or more central atoms in a symmetrical manner. The peripheral atoms are usually one or more of molybdenum, tungsten, vanadium, niobium, tantalum and other metals. The central atoms are usually silicon or phosphorus but can comprise any one of a large variety of atoms from Groups I-VIII in the Periodic Table of elements. These include, for instance, cupric ions; divalent beryllium, zinc, cobalt or nickel ions; trivalent boron, aluminium, gallium, iron, cerium, arsenic, antimony, phosphorus, bismuth, chromium or rhodium ions; tetravalent silicon, germanium, tin, titanium, zirconium, vanadium, sulphur, tellurium, manganese nickel, platinum, thorium, hafnium, cerium ions and other rare earth ions; pentavalent phosphorus, arsenic, vanadium, antimony ions; hexavalent tellurium ions; and heptavalent iodine ions. Such heteropolyacids are also known as "polyoxoanions", "polyoxometallates" or "metal oxide clusters". The structures of some of the well known anions are named after the original researchers in this field and are known e.g. as Keggin, Wells-Dawson and Anderson-Evans-Perloff structures.

Heteropolyacids usually have a high molecular weight e.g. in the range from 700-8500 and include dimeric complexes.

When used in the form of salts, the solubility of the salts can be controlled by choosing the appropriate counterions. Preferably, however, the heteropolyacid in the present invention is in the form of the free acid (or at least predominantly in the form of the free acid, by which is meant that at least 50% of the heteropolyacid present is in the form of the free acid).

Specific examples of heteropolyacids that may be used in the present invention include tungstophosphonic acids, tungstosilicic acids, molybdophosphonic acids, and molybdosilicic acids, and their corresponding salts (and which, for avoidance of doubt, includes their compositional analogues, such as tungstodiphosphonic acid and its salts).

The heteropolyacid is used on a support at an acid loading of 0.5 to 0.9 mg/m² of catalyst support surface area.

Preferably the acid loading is at least 0.6 mg/m² such as at least 0.7 mg/m² of catalyst support surface area.

Preferably the acid loading is less than 0.85 mg/m², such as less than 0.8mg/m², of catalyst support surface area.

Catalyst support surface area, as used herein, refers to BET surface area, as measured according to ASTM D 3663-03, with measurement of 50 points on the adsorption and desorption isotherms, and a degassing temperature of 250°C.

As noted above, it has now been found that significant advantages are obtained in alcohol dehydration by supported heteropolyacid catalysts by selection of the specific catalyst loadings. In particular, it has surprisingly been found that over a relatively narrow range of loadings improved ethylene productivity and reduced carbon formation on the catalyst is obtained.

Any suitable support may be used, but preferred supports are inorganic oxides, such as silica, alumina, titania, zirconia, with silica being the most preferred.

Preferred silica supports may be porous synthetic silicas in the form of extrudates or pellets, for example as described in EP 0 704 240 A1.

The support is suitably in the form of particles having a diameter of 2 to 10 mm, preferably 4 to 6 mm. The support suitably has a pore volume in the range from 0.3-1.2 ml/g, preferably from 0.6-1.0 ml/g. The support suitably has a crush strength of at least 2 Kg force, suitably at least 5 Kg force, preferably at least 6 Kg and more preferably at least 7 Kg. The crush strengths quoted are based on average of that determined for each set of 50 particles on a CHATTILLON tester which measures the minimum force necessary to crush a particle between parallel plates.

The support suitably has an average pore radius (prior to use) of 10 to 500 Angstroms, preferably an average pore radius of 40 to 180 Angstroms.

An example of a particularly preferred support is Grace 57 silica.

Grace 57 silica has a bulk density of approximately 0.4 g/l and a surface area of approximately 315 m²/g, and thus the range 0.5 to 0.9 mg/m² corresponds to an approximate loading of 13 to 22 wt% of the heteropolyacid based on the total weight of heteropolyacid and support.

In one embodiment, therefore, the supported heteropolyacid catalyst used for the present invention preferably has a heteropolyacid loading comprising 13-22 wt% of the heteropolyacid based on the total weight of heteropolyacid and support, and more preferably comprises 15 to 21 wt% of heteropolyacid based on the total weight of heteropolyacid and support.

In order to achieve optimum performance, the support is suitably free of extraneous metals or elements which might adversely affect the catalytic activity of the system.

The synthesis of heteropolyacids, and their impregnation onto supports where required, may be performed by any known methods, such as those described in EP 0 704 240 A1, EP 1 140 703 B1, WO 2007/003901 and North, E. O.; "Organic Synthesis" 1 p. 129 (1978), published by Huntington N.Y.

If required the heteropolyacid may be purified, for example by using liquid/liquid extraction with a suitable solvent, such as described in EP 1 140 703 B1.

The reaction of the process of the present invention may be performed under any suitable conditions.

Preferably, however, the reaction is performed by passing the one or more alcohols in the vapour phase over a bed of the supported heteropolyacid catalyst.

Relatively high temperatures are preferred in the process of the present invention, especially in the range 175°C to 300°C. More preferably the temperature is above 200 °C and/or less than 280°C.

In a particular embodiment, the temperature is at least 230°C, for example 230°C to 280°C.

It has been found that a relatively low level of alkane formation compared to the desired alkene is obtained when reacting the catalyst of the present invention at relatively high temperatures, even in the presence of ethers in the feed.

As a general rule, ethers in the feed can promote formation of alkanes. Thus, reacting a feed comprising ethanol without diethyl ether being present results in relatively low formation of ethane in ethylene whereas reaction of a feed which is essentially diethyl ether and water in the absence of ethanol will still produce ethylene but will result in the formation of large quantities of ethane in the produced ethylene. Contrary to what is suggested from these results it has been found that significant quantities of ethers can be tolerated in the feed comprising one or more alcohols according to the present invention without significantly increased alkane make compared to absence of the ethers.

The amount of ethers that can be tolerated without significantly increased alkane make can be higher, but typically the relative weight of ethers to alcohols in the feed to the process of the present invention is less than 2:1, especially less than 60wt% ethers and at least 30wt% alcohols.

This tolerance to ethers and reduced alkane make gives significant process advantages. Firstly, this results in a product stream which requires less purification of the desired olefins in the first place, simplifying downstream processing. Secondly, the process of the present invention usually produces a product stream comprising the desired olefins (dehydration product), unreacted alcohols and ethers, and the unreacted alcohols and ethers can be recycled to the process without significantly affecting the product quality.

In addition, the use of relatively high temperatures can provide improved selectivity to alkene compared to ethers in the first place, minimizing the quantity of ethers formed and subsequently required to be recycled.

The reaction may be performed at atmospheric or elevated pressure. Preferably, the reaction is performed at elevated pressure, especially at least 5 barg, more preferably at least 10 barg. Preferably, the pressure is less than 40 barg.

The reaction is performed at any suitable gas hourly space velocity. Preferably, the reaction is performed at a liquid hourly space velocity (LHSV) in the range 1 to 10 (ml feed/ml catalyst/hour).

### Examples

### Catalyst Preparation

12-tungstosilicic acid.xH2O (H4SiW12O40.xH2O, x being about 24) was used as Acid A.

For catalyst preparation using Acid A, samples of Acid A were dissolved in distilled water to which was added orthophosphoric acid. Grace 57 silica (specific surface area of 310.5 m²/g) was then added in the amounts required for the desired acid loadings. Each sample was allowed to soak for 24 hours, and after soaking the catalyst was drained of excess solution for 1 hour and then dried in air for 16 hours at 105°C.

For K modification, samples of Acid A were dissolved in distilled water to which was added orthophosphoric acid. In a separate container KHCO3 was dissolved in water and then slowly added, with stirring, to the acid solution. (The quantity of KHCO3 was chosen so as to provide one molar equivalent of potassium per mole of 12-tungstosilicic acid.xH2O dissolved in the solution). The above solution was stirred for 15 minutes after the evolution of CO2 had ceased. This is used as Acid B.

For catalyst preparation using Acid B, samples of the above solution of Acid B were added to Grace 57 silica in the amounts required for the desired acid loadings. Each sample was allowed to soak for 24 hours, and after soaking the catalyst was drained of excess solution for 1 hour and then dried in air for 16 hours at 105°C.

Catalysts were prepared as follows:

| Catalyst Number | Acid | Acid loading (mg/m²) |
|---|---|---|
| 1 | A | 0.50 |
| 2 | A | 0.62 |
| 3 | A | 0.77 |
| 4 | A | 0.92 |
| 5 | B | 0.51 |
| 6 | B | 0.61 |
| 7 | B | 0.77 |
| 8 | B | 0.90 |
| 9 | B | 1.09 |

### Catalyst Testing

### Atmospheric pressure tests

Catalysts were screened at atmospheric pressure.

A feed comprising 10% ethanol in helium was passed in the vapour phase over the catalyst for a period of 12 hours at a temperature of 210°C, and then replaced with a feed comprising 5% diethyl ether (DEE) in helium for a period of 5 days, again at 210°C. The feed was then returned to 10% ethanol in helium for 12 hours at 210°C, followed by a final 12 hours at 190°C. A liquid hourly space velocity of 1.3 ml/ml/hr was used throughout.

### Elevated pressure tests

Several catalysts were also tested at elevated pressure.

The test equipment for elevated pressure tests consisted of a Hastelloy fixed bed reactor, which was electrically heated via two independently controlled heating zones. A charge of 50ml catalyst was loaded into the reactor such that it was entirely contained within the heated zones. Liquids were fed to the reactor via a constametric HPLC pump via a trace heated line, entering the reactor as a vapour. Liquid products were collected in a catch-pot and gaseous products passed to a wet gas meter to measure the volume of gas generated. Analysis of liquid and gaseous samples was via Gas Liquid Chromatography for organic components, and Karl Fischer titration for water content.

A feed comprising 58wt% ethanol, 38wt% diethyl ether (DEE) and 4wt% water (which represents a feed for a process with recycle of ethers and unreacted alcohol) was passed in the vapour phase to the reactor at a pressure of 20 barg and liquid hourly space velocities of 1-9 ml/ml/hr. Experiments were performed at reactor inlet temperature of 200-260°C over a period of at least 300 hours.

### Results

### Atmospheric pressure tests

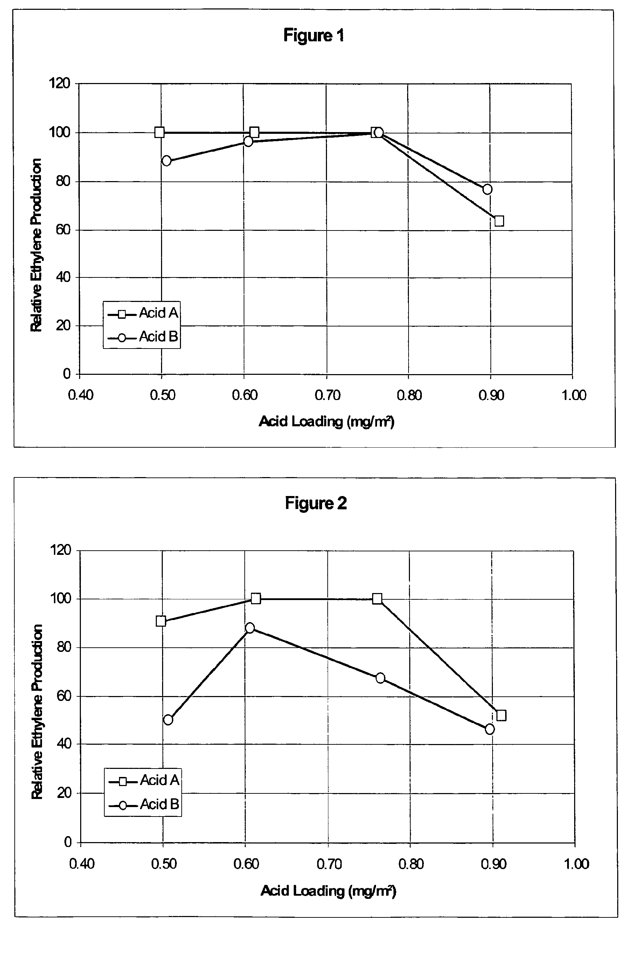

Results for ethylene production from the feed comprising 10% ethanol in helium are shown in Figure 1 after the first 12 hours and in Figure 2 after 5 days. (Both graphs and both catalysts are normalised to 100 based on the maximum ethylene production from catalysts with Acid A in Figure 1.) It can be seen that an optimum in ethylene productivity is observed within the acid loading range of the present invention, the optimum being particularly pronounced after the longer time on stream shown in Figure 2, showing both an improvement in ethylene productivity and a reduced deactivation rate within the range of the present invention.

The graphs also show a surprising improvement in catalyst stability when using a supported tungstosilicic acid with no cation modification (Acid A). In particular, catalysts of Acid A do not deactivate to any measurable extent for HPA loadings of 0.62 and 0.77 mg/m².

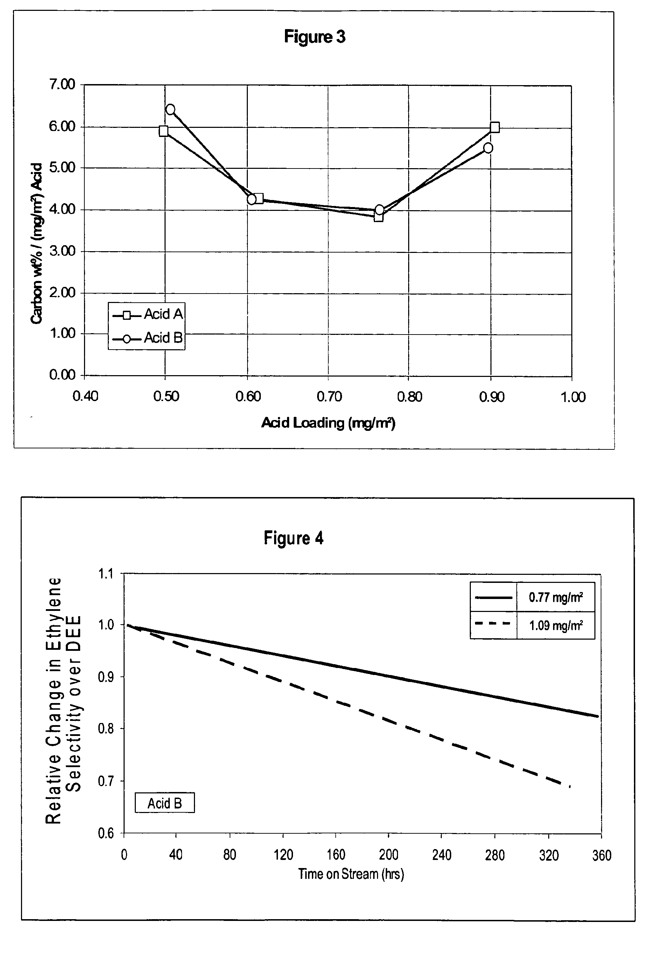

Figure 3 represents carbon analysis results from the catalysts after removal from the reactor. Reduced carbon lay-down is observed within the range of acid loading of the present invention, (with a minimum between 0.5 to 0.9 mg/m² of catalyst support surface area) consistent with the reduced deactivation rate shown by comparison between Figures 1 and 2.

### Elevated pressure tests

Results for ethylene selectivity versus time on stream are shown in Figure 4 for two catalysts with Acid B at different loadings. The results are normalised to highlight the differences in the relative rate of deactivation. It can be seen that the catalyst with a loading of 0.77 mg/m² i.e. within the range of the present invention, (catalyst 8) shows significantly reduced deactivation rate (in terms of selectivity loss) than the catalyst with higher acid loading (catalyst 9).

The catalyst according to the present invention also produced a product with relatively low ethane impurity in the produced ethylene. In particular, the ethane in ethylene was below 500ppm throughout the 360 hours of the experiment.

### Effect of diethyl ether co-feed

Experiments were performed as for the elevated pressure tests detailed above, except that three experiments were performed with different feeds over the same catalyst at 240°C to investigate the effect of the feed composition on the ethane impurity in the ethylene produced.

Feed 1 was a feed comprising essentially diethylether, comprising 97wt% diethyl ether and 3wt% water.

Feed 2 had no diethylether and comprised 95.4wt% ethanol and 4.6wt% water.

Feed 3 comprised 58wt% ethanol, 38wt% diethyl ether (DEE) and 4wt% water (which represents a potential feed for a process with recycle of ethers and unreacted alcohol).

Feed 1 resulted in the formation of 1169 ppm ethane in ethylene and feed 2 resulted in the formation of 466 ppm ethane in ethylene. Comparison of these results shows that reaction of diethyl ether results in significantly increased formation of ethane in the product ethylene. Feed 3, however, resulted in only 463 ppm of ethane in ethylene despite the presence of significant quantities of diethyl ether in the feed. Contrary to what is suggested from the result using diethyl ether and water only (Feed 1), therefore, these examples show that nearly 40wt% of diethyl ether can be tolerated without significantly increased alkane make compared to absence of diethyl ether.

## Claims

1. A process for the dehydration of one or more alcohols, which process comprises contacting one or more alcohols with a supported heteropolyacid catalyst, said supported heteropolyacid catalyst having a heteropolyacid loading of from 0.5 to 0.9 mg/m² of catalyst support surface area.

2. A process as claimed in claim 1 wherein the one or more alcohols is a mixture of alcohols comprising ethanol and propanol.

3. A process as claimed in claim 1 or claim 2 wherein the heteropolyacid is selected from tungstophosphonic acids, tungstosilicic acids, molybdophosphonic acids, and molybdosilicic acids, and their corresponding salts.

4. A process as claimed in any one of the preceding claims wherein the heteropolyacid is used on a support at an acid loading of 13 to 22 wt% of the heteropolyacid based on the total weight of heteropolyacid and support

5. A process as claimed in any one of the preceding claims wherein the heteropolyacid is in the form of the free acid.

6. A process as claimed in any one of the preceding claims wherein the heteropolyacid is used on a silica support.

7. A process as claimed in any one of the preceding claims which comprises passing the one or more alcohols in the vapour phase over a bed of the supported heteropolyacid catalyst and at a temperature in the range 175°C to 300°C.
